# EUROPÄISCHE PATENTSCHRIFT

(11) **EP 0 683 154 B1**
(45) Veröffentlichungstag und Bekanntmachung des Hinweises auf die Patenterteilung: **19.08.1998**
(21) Anmeldenummer: 95106729.7
(22) Anmeldetag: 04.05.1995
(51) Int. Cl.: C07C 265/14

(54) **Neue Diisocyanate, ein Verfahren zu ihrer Herstellung und ihre Verwendung**
Diisocyanates, process for their preparation and their use
Diisocyanates, procédé pour leur préparation et leur utilisation

(30) Priorität: 17.05.1994 DE 4417186
(43) Veröffentlichungstag der Anmeldung: 22.11.1995
(73) Patentinhaber: BAYER AG, 51368 Leverkusen (DE)
(72) Erfinder: Wamprecht, Christian, Dr., D-41472 Neuss (DE); Jost, Klaus, Dr., D-41539 Dormagen (DE); Penninger, Stefan, Dr., D-50259 Pulheim (DE)

(56) Entgegenhaltungen:
- EP-A- 0 157 661
- DE-A- 1 418 995
- US-A- 4 224 238

## Beschreibung

Gegenstand der vorliegenden Erfindung sind neue Diisocyanate der Formel 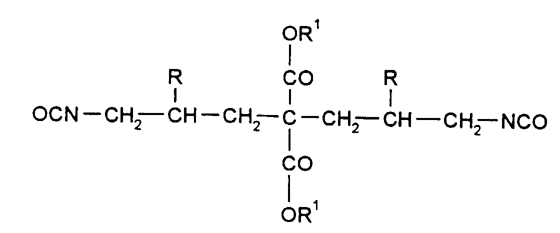 in welcher
- R: für gleiche oder verschiedene Reste stehen und Wasserstoff oder einen aliphatischen Kohlenwasserstoffrest mit 1 bis 4 Kohlenstoffatomen bedeuten und
- R¹: für gleiche oder verschiedene Reste stehen und einen aliphatischen, araliphatischen und/oder cycloaliphatischen Rest mit 1 bis 12 Kohlenstoffatomen bedeuten.

Gegenstand der Erfindung ist auch ein Verfahren zur Herstellung dieser Diisocyanate, welches dadurch gekennzeichnet ist, daß man, gegebenenfalls in der Ammoniumsalz-Form vorliegende Diamine der Formel 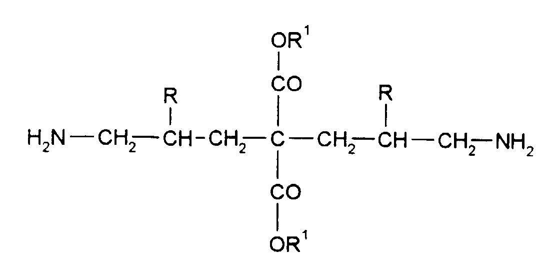 in an sich bekannter Weise phosgeniert.

Bei den erfindungsgemäßen Verbindungen handelt es sich um neuartige, Carbonsäureestergruppen aufweisende Diisocyanate, die eine Reihe wertvoller Eigenschaften in sich vereinigen:
1. Die neuen Diisocyanate weisen aliphatisch gebundene Isocyanatgruppen auf, so daß sie insbesondere zur Herstellung von lichtechten, witterungsbeständigen, Urethan- und/oder Harnstoffgruppen aufweisenden Kunststoffen bzw. von Vorprodukten zur Herstellung derartiger Kunststoffe geeignet sind.
2. Die neuen Diisocyanate stellen niedrigviskose, hochsiedende Flüssigkeiten oder Feststoffe mit niedrigem Schmelzpunkt dar und sind daher den bekannten aliphatischen Diisocyanaten mit weit höherem Dampfdruck, wie z.B. Hexamethylendiisocyanat, in physiologischer Hinsicht überlegen.
3. Die neuen Diisocyanate weisen Carbonsäureestergruppen auf, was eine breite Variationsmöglichkeit bezüglich ihrer Eigenschaften wie z.B. ihrer Viskosität, ihrer Löslichkeit in organischen Lösungsmitteln oder ihrer Verträglichkeit mit Reaktionspartnern durch Variation der inerten Reste R und insbesondere R¹ eröffnet.

Die Herstellung der erfindungsgemäßen Diisocyanate geschieht durch an sich bekannte Phosgenierung der ihnen zugrundeliegenden, gegebenenfalls in der Ammoniumsalz-Form, insbesondere als Dihydrochloride vorliegenden Diamine der Formel 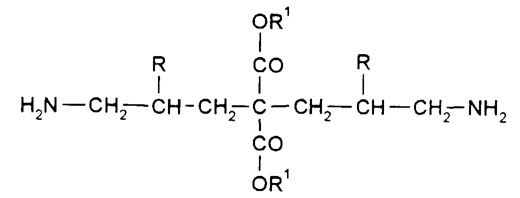 hier, vor- und nachstehend haben die Reste R und R¹ die vorstehend genannte Bedeutung, wobei R vorzugsweise für Wasserstoff oder eine Methylgruppe und R¹ vorzugsweise für eine Methyl- oder Ethylgruppe stehen.

Die Herstellung der beim erfindungsgemäßen Verfahren einzusetzenden Diamine kann beispielsweise in einer zweistufigen Reaktion erfolgen:

In einer ersten Reaktionsstufe werden, wie beispielsweise in "Organikum", 15. Auflage, VEB Deutscher Verlag der Wissenschaften, Berlin 1977 auf Seiten 633 ff. beschrieben, 2 Mol eines Nitrils der Formel 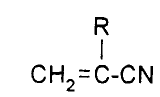 mit 1 Mol eines Malonsäureesters der Formel

R¹OOC-CH₂-COOR¹

zu einem Dinitril der Formel 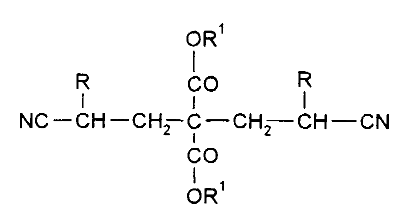 umgesetzt. Diese an sich bekannte Reaktion kann beispielsweise bei 0 bis 80°C, vorzugsweise bei 30 bis 45°C in Gegenwart von 1 bis 5 Mol-%, bezogen auf die Menge des eingesetzten Malonsäureesters, einer starken Base wie beispielsweise Natrium- oder Kaliumhydroxid, gegebenenfalls in Anwesenheit eines alkoholischen Lösungsmittels vorgenommen werden. Oftmals wird eine geringe Menge eines geeigneten Alkohols, der vorzugsweise dem Rest des eingesetzten Malonsäureesters entspricht, als Lösungsmittel für die eingesetzte Base mitverwendet.

Geeignete Nitrile sind beispielsweise Acrylnitril, Methacrylnitril, 2-Ethyl-acrylnitril, 2-Propyl-acrylnitril oder 2-Butyl-acrylnitril. Bevorzugt sind Acrylnitril und Methacrylnitril. Besonders bevorzugt ist Acrylnitril.

Geeignete Malonsäureester sind beispielsweise Malonsäuredimethylester, Malonsäurediethylester, Malonsäuredi-n-propylester, Malonsäurediisopropylester, Malonsäuredi-n-butylester, Malonsäurediisopropylester, Malonsäuredi-tert.-butylester, Malonsäuredi-n-hexylester, Malonsäuredi-2-ethylhexylester, Malonsäuredibenzylester oder Malonsäuredicyclohexylester. Bevorzugt sind Malonsäuredimethylester und Malonsäurediethylester.

Die Überführung der genannten Dinitrile in die Diamine geschieht in der zweiten Stufe der Herstellung der erfindungsgemäß einzusetzenden Ausgangsmaterialien durch katalytische Hydrierung, beispielsweise in Gegenwart von schwach polaren bzw. unpolaren organischen Lösungsmitteln und Ammoniak, bei 80 bis 130°C und einem Druck von 120 bis 200 bar, wobei Raney-Kobalt oder Raney-Nickel geeignete Katalysatoren sind, oder in Gegenwart von Carbonsäure als Lösungsmittel und Chlorwasserstoff oder einer anderen, zur Ammoniumsalzbildung mit den entstehenden Diaminen befähigten Säure, bei 10 bis 100°C und einem Druck von 20 bis 150 bar, wobei Katalysatoren auf Basis von Platin oder Palladium, wie z.B. die genannten Metalle oder deren Oxide, geeignet sind. Beim Arbeiten in saurem Medium fallen die Diamine als entsprechende Ammoniumsalze an. Bevorzugt wird die Hydrierung in saurem Medium durchgeführt.

Zur Hydrierung werden die Dinitrile vorzugsweise in 5 bis 40 gew.-%iger Lösung in Lösungsmitteln wie z.B. Methanol, Ethanol, Isopropanol, Dioxan, Tetrahydrofuran oder deren Gemischen beim Arbeiten in alkalischem Medium (Ammoniak) oder Ameisensäure, Essigsäure, Propionsäure, Chloressigsäure oder deren Gemischen beim Arbeiten in saurem Medium gelöst.

Falls die Hydrierung in Gegenwart von starken, zur Ammoniumsalzbildung befähigten Säuren durchgeführt wird, fallen die Diamine unmittelbar als Ammoniumsalze an. Hierbei können statt des beispielhaft genannten Chlorwasserstoffs auch andere Säuren wie beispielsweise Bromwasserstoff, Schwefelsäure oder Phosphorsäure eingesetzt werden, obwohl Chlorwasserstoff bevorzugt ist.

Die bei der Hydrierung erhaltenen Lösungen der erfindungsgemäßen Diamine können mittels Filtration vom Katalysator, mittels Destillation vom Lösungsmittel befreit und anschließend gelöst in einem für die Phosgenierung geeigneten Lösungsmittel ohne weitere Aufarbeitung dem Phosgenierungsprozeß zugeführt werden. Es ist jedoch auch möglich, die Diamine, insbesondere entsprechende Diaminhydrochloride in reiner Form zu isolieren und anschließend der Phosgenierungsreaktion zuzuführen.

Zur Durchführung des erfindungsgemäßen Phosgenierverfahrens werden die Diamine bzw. Diaminhydrochloride in an sich bekannter Weise, beispielsweise nach dem Kalt-Heiß-Phosgenierverfahren (W. Siefken, Annalen der Chemie, 562, (1949), Seiten 75 ff.) phosgeniert und destillativ aufgearbeitet. Vorzugsweise geschieht die Phosgenierung in Gegenwart eines geeigneten Lösungsmittels wie z.B. Chlorbenzol oder o-Dichlorbenzol.

Die erfindungsgemäßen Diisocyanate stellen einen neuen Typ aliphatischer Diisocyanate dar, die insbesondere durch die als Substituenten fungierenden Carbonsäureestergruppen gekennzeichnet sind.

Aus der US-A 4224238, DE-A 1418995 und EP-A 157661 sind Diisocyanate bekannt, die Carbonestergruppen und /oder Carboxylgruppen im Molekül tragen können, die α-ständig zur NCO-Gruppe sind. Über deren Verwendung werden jedoch keine Angaben gemacht.

Die erfindungsgemäßen Diisocyanate stellen wertvolle Ausgangsmaterialien zur Herstellung von Urethan- und/oder Harnstoffgruppen aufweisenden Kunststoffen nach dem Isocyanat-Polyadditionsverfahren dar. Hierbei können sie anstelle oder in Kombination mit den bekannten Polyisocyanaten der Polyurethanchemie in an sich bekannter Weise mit den üblichen Verbindungen mit gegenüber Isocyanatgruppen reaktionsfähigen Hydroxyl- und/oder Aminogruppen zur Reaktion gebracht werden Die erfindungsgemäßen Diisocyanate eignen sich jedoch auch zur Herstellung von Vorprodukten zur Herstellung derartiger Kunststoffe, d.h. insbesondere zur Herstellung von modifizierten Polyisocyanaten, die ihrerseits Ausgangsmaterialien zur Herstellung von Kunststoffen der genannten Art darstellen. Modifizierte Polyisocyanate sind beispielsweise die an sich bekannten Allophanat-, Biuret-, Isocyanurat-, Urethan- und/oder Uretdiongruppen aufweisenden Polyisocyanate, wie sie bislang insbesondere auf Basis von Hexamethylendiisocyanat und/oder von Isophorondiisocyanat hergestellt worden sind und Eingang in die Praxis gefunden haben. Bei der Herstellung von derartigen modifizierten Polyisocyanaten auf Basis der erfindungsgemäßen Diisocyanate werden diese anstelle oder in Abmischung mit den bislang hierzu eingesetzten Diisocyanaten des Standes der Technik bei den an sich bekannten Modifizierungsverfahren eingesetzt. Die erfindungsgemäßen Diisocyanate und ihre Isocyanatgruppen aufweisenden Modifizierungsprodukte eignen sich insbesondere als Polyisocyanatkomponente für Polyurethan-Klebstoffe und -Beschichtungsmassen.

In den nachfolgenden Beispielen beziehen sich alle Prozentangaben auf das Gewicht. Die "HC-Werte" geben den Gehalt an hydrolysierbarem Chlor an.

### Beispiel 1

### a) Di-(2-cyanoethyl)malonsäurediethylester

Zu einer Mischung aus 1920 g Malonsäurediethylester (12 Mol), 12 g Kaliumhydroxid und 120 g Ethanol wurden unter Rühren bei Raumtemperatur 1399 g Acrylnitril (26,4 Mol) so zugegeben, daß die Temperatur, gegebenenfalls durch Kühlen, bei 35 bis 40°C gehalten werden konnte. Nach beendeter Zugabe blieb der Ansatz ohne Rühren über Nacht stehen. Der entstandene Kristallbrei wurde mit Wasser verrührt, abgesaugt und aus Ethanol umkristallisiert. Es wurden 2947 g (92,3 % der Theorie) farblose Kristalle vom Schmelzpunkt 61,7 % erhalten.

### b) Di-(3-aminopropyl)malonsäurediethylesterdihydrochlorid

In einem 1,3 l Rührautoklaven aus einer korrosionsfesten Metallegierung wurden 100 g Di-(2-cyanoethyl)malonsäurediethylester, 800 ml Eisessig und 10 g Platinoxid vorgelegt. Nach dreimaligem Spülen mit Stickstoff wurden 28 g Chlorwasserstoff eingeleitet, 40 bar Wasserstoff aufgedrückt und so lange bei 20 bis 40°C hydriert, bis Druckkonstanz erreicht war. Anschließend wurde 1 Stunde bei 35°C und einem Wasserstoffdruck von 100 bar gerührt. Der Autoklav wurde anschließend entspannt, entleert und mit Eisessig gespült.

Die Produktlösungen von 10 Autoklaven-Ansätzen wurden über eine Drucknutsche filtriert und das Lösungsmittel des Filtrats am Rotationsverdampfer bei maximal 100°C abdestilliert. Das anfallende, Eisessig enthaltende, zähelastische Produkt wurde mit Chlorbenzol verrührt, wobei Kristallisation eintrat. Der Kristallbrei wurde abgesaugt, mit Aceton kurz gewaschen und trockengesaugt. Nach 20-stündigem Trocknen im Vakuumtrockenschrank bei ca. 90 bis 100°C und 15 mbar fiel das Produkt als nahezu farblose, pulverförmige Substanz vom Schmelzpunkt 190°C an. Ausbeute 1210 g (94 % der Theorie).

IR-, ¹H-NMR und ¹³C-NMR-Spektren bestätigen die angegebene Struktur.

### c) Di-(3-isocyanatopropyl)malonsäurediethylester

### c1) Phosgenierung und Lösungsmittelabtrennung

In eine Suspension von 62,3 g Di-(3-aminopropyl)malonsäurediethylesterdihydrochlorid in 1,5 l Chlorbenzol wurde unter Rückfluß bis zum Klarpunkt gasförmiges Phosgen eingeleitet. Anschließend wurde mit Stickstoff entphosgeniert und dann das Lösungsmittel im Vakuum abdestilliert. 58,2 g Di-(3-isocyanatopropyl)malonsäurediethylester wurden als Rohprodukt erhalten. NCO-Gehalt: 24,2 % (Theorie: 25,8 %).

### c2) Temperung und Destillation

In einem 500 ml Rundkolben mit Claisenbrücke wurden 420 g Di-(3-isocyanatopropyl)malonsäurediethylester (Rohprodukt) 25 Minuten bei ca. 180°C und ca. 0,3 mbar getempert und anschließend einer Flashdestillation unterzogen (25 g Sumpf). Der HC-Wert betrug 950 ppm.

Zur Feindestillation wurden 355 g Diisocyanat in einem Zweihalskolben mit Vigreuxkolonne, Liebigkühler und Vakuumwechselvorlage eingesetzt. Bei einer Destillationsgeschwindigkeit von ca. 1 Tropfen/Sekunde wurden 5 g Vorlauf entnommen und das Produkt bei einer Kopftemperatur von 164°C bei einem Druck von 0,1 mbar überdestilliert (6 g Sumpf). Die Ausbeute an farbloser Flüssigkeit betrug 342 g (96 % der Theorie). NCO-Gehalt: 24,9 % (Theorie: 25,8 %), HC-Wert: 232 ppm.

IR-, ¹H-NMR-, ¹³C-NMR-, Massenspektrum sowie die chemische Analyse bestätigen die angegebene Konstitution.

| | | | | |
|---|---|---|---|---|
| C₁₅H₂₂N₂O₆ (326) | Ber.: | C: 55,2 % | H: 6,75 % | N: 8,59 % |
| | Bef.: | C: 55,4 % | H: 6,99 % | N: 8,76 % |

### Beispiel 2

### a) Di-(2-cyanoethyl)malonsäuredimethylester

Zu einer Mischung aus 792 g Malonsäuredimethylester (6 Mol), 6 g Kaliumhydroxid und 60 g Ethanol wurden unter Rühren bei Raumtemperatur 700 g Acrylnitril (13,2 Mol) so zugetropft, daß die Temperatur, gegebenenfalls durch Kühlen, bei 35 bis 40°C gehalten werden konnte. Nach beendeter Zugabe blieb der Ansatz ohne Rühren über Nacht stehen. Der entstandene Kristallbrei wurde mit Wasser/Ethanol verrührt, abgesaugt und getrocknet. Es wurden 1173 g (82 % der Theorie) farblose Kristalle vom Schmelzpunkt 143°C erhalten.

### b) Di-(3-aminopropyl)malonsäuredimethylesterdihydrochlorid

Analog dem Verfahren gemäß Beispiel 1b) wurden 100 g Di-(2-cyanoethyl)malonsäuredimethylester hydriert. Es wurden nach Aufarbeitung von 10 Autoklavenansätzen 1240 g nahezu farblose Kristalle (92,8 % der Theorie) vom Schmelzpunkt 210°C (Zersetzung) erhalten.

IR-, ¹H-NMR- und ¹³C-NMR-Spektrum bestätigen die angegebene Struktur.

### c) Di-(3-isocyanatopropyl)malonsäuredimethylester

### cl) Phosgenierung und Lösungsmittelabtrennung

In eine Suspension von 60,5 g Di-(3-aminopropyl)malonsäuredimethylesterdihydrochlorid in 1,5 o-Dichlorbenzol wurde unter Rückfluß bis zum Klarpunkt gasförmiges Phosgen eingeleitet. Anschließend wurde mit Stickstoff entphosgeniert und dann das Lösungsmittel im Vakuum abdestilliert. 56,6 g Di-(3-isocyanatopropyl)malonsäuredimethylester wurden als Rohprodukt erhalten. NCO-Gehalt: 29,6 % (Theorie: 28,19 %).

### c2) Temperung und Destillation

In einen 500 ml Rundkolben mit Claisenbrücke wurden 400 g Di-(3-isocyanatopropyl)malonsäuredimethylester (Rohprodukt) 40 Minuten bei ca. 185°C und ca. 1,5 mbar getempert und anschließend einer Flashdestillation unterzogen (20 g Sumpf). Der HC-Wert betrug 640 ppm.

Zur Feindestillation wurden 350 g Diisocyanat in einem 500 ml Zweihalskolben mit Vigreuxkolonne, Liebigkühler und Vakuumwechselvorlage eingesetzt. Bei einer Destillationsgeschwindigkeit von ca. 1 Tropfen/Sekunde wurden 6 g Vorlauf entnommen und das Produkt bei einer Kopftemperatur von 180°C bei einem Druck von 0,7 mbar überdestilliert (8 g Sumpf). Die Ausbeute an farbloser Flüssigkeit betrug 336 g (96 % der Theorie).
NCO-Gehalt: 27,45 % (Theorie: 28,19 %), HC-Wert: 248 ppm.

IR-, ¹H-NMR-, ¹³C-NMR-, Massenspektrum sowie die chemische Analyse bestätigen die angegebene Konstitution.

| | | | | |
|---|---|---|---|---|
| C₁₃H₁₈N₂O₆ (298) | Ber.: | C: 55,3 % | H: 6,04 % | N: 9,40 % |
| | Bef.: | C: 52,4 % | H: 5,99 % | N: 9,51 % |

## Patentansprüche

1. Diisocyanate der Formel 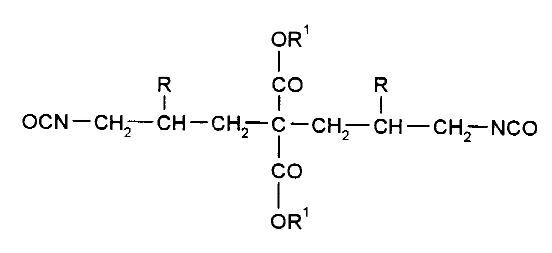 in welcher
R für gleiche oder verschiedene Reste stehen und Wasserstoff oder einen aliphatischen Kohlenwasserstoffrest mit 1 bis 4 Kohlenstoffatomen bedeuten und
R¹ für gleiche oder verschiedene Reste stehen und einen aliphatischen, araliphatischen und/oder cycloaliphatischen Rest mit 1 bis 12 Kohlenstoffatomen bedeuten.

2. Diisocyanate gemäß Anspruch 1, dadurch gekennzeichnet, daß R für Wasserstoff oder eine Methylgruppe und R¹ für eine Methyl- oder Ethylgruppe steht.

3. Verfahren zur Herstellung von Diisocyanaten gemäß Anspruch 1, dadurch gekennzeichnet, daß man, gegebenenfalls in der Ammoniumsalz-Form vorliegende Diamine der Formel 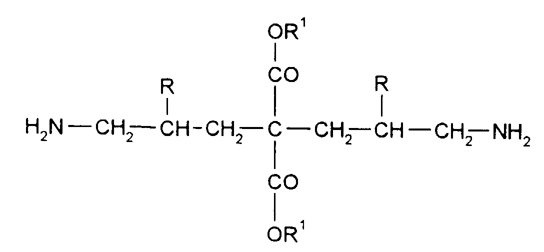 in an sich bekannter Weise phosgeniert.

4. Verfahren gemäß Anspruch 3, dadurch gekennzeichnet, daß man die entsprechenden Diamindihydrochloride phosgeniert.

5. Verwendung der Diisocyanate gemäß Anspruch 1 und 2 als Ausgangsmaterial bei der Herstellung von Urethan- und/oder Harnstoffgruppen aufweisenden Kunststoffen oder als Ausgangsmaterial zur Herstellung von Vorprodukten zur Herstellung derartiger Kunststoffe.

## Claims

1. Diisocyanates of the formula: 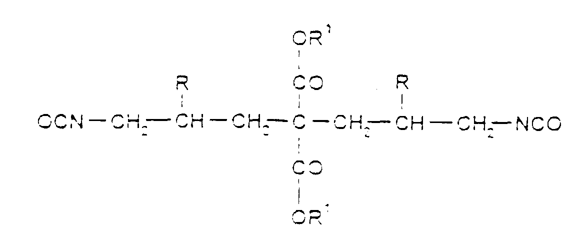 in which
R stands for radicals which may be the same or different and denotes hydrogen or an aliphatic hydrocarbon radical having 1 to 4 carbon atoms, and
R¹ stands for radicals which may be the same or different and denotes an aliphatic, araliphatic and/or cycloaliphatic radical having 1 to 12 carbon atoms.

2. Diisocyanates according to Claim 1, characterised in that R stands for hydrogen or a methyl group and R¹ stands for a methyl or ethyl group.

3. Process for the preparation of diisocyanates according to Claim 1, characterised in that diamines of the formula: 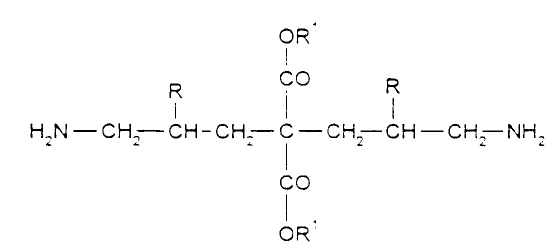 which are present optionally in the ammonium salt form are phosgenated in a manner known per se.

4. Process according to Claim 3, characterised in that the corresponding diamine dihydrochlorides are phosgenated.

5. Use of the diisocyanates according to Claims 1 and 2 as a starting material in the preparation of plastics materials which contain urethane and/or urea groups or as a starting material of the preparation of intermediate products for the preparation of such plastics materials.

## Revendications

1. Diisocyanates de la formule 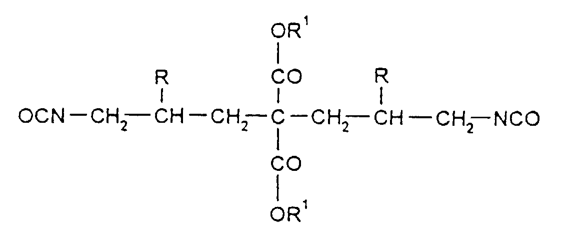 dans laquelle
R représente des radicaux identiques ou différents et l'hydrogène, ou un radical d'hydrocarbure aliphatique comportant 1 à 4 atomes de carbone et
R¹ correspond à des radicaux identiques ou différents et à un radical aliphatique, araliphatique et/ou cycloaliphatique possédant 1 à 12 atomes de carbone.

2. Diisocyanates conformes à la revendication 1, caractérisés en ce que R représente l'hydrogène ou un groupe méthyle et R¹ correspond à un groupe méthyle ou éthyle.

3. Procédé de préparation de diisocyanates conformes à la revendication 1, caractérisé en ce que l'on phosgénise d'une manière connue en soi des diamines éventuellement présentes sous la forme de sel d'ammonium, de la formule

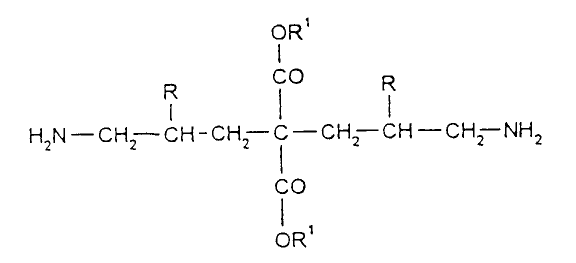

4. Procédé selon la revendication 3, caractérisé en ce que l'on phosgénise les chlorhydrates de diamine correspondants.

5. Utilisation des diisocyanates conformes aux revendications 1 et 2, comme matière de départ pour la préparation de matières synthétiques présentant des groupes uréthane et/ou urée, ou comme matière de départ pour la préparation de précurseurs de matières synthétiques de cette espèce.
